# EUROPEAN PATENT APPLICATION

(11) **EP 3 170 895 A1**
(43) Date of publication of application: **24.05.2017**
(21) Application number: 15822337.0
(22) Date of filing: 17.07.2015
(51) Int. Cl.: C12N 5/0783, A61K 35/17, A61P 31/12, A61P 35/00, A61P 37/02, A61P 37/08, C12N 5/095, C12N 15/09

(54) **PRODUCTION METHOD FOR PLURIPOTENT STEM CELLS HAVING ANTIGEN-SPECIFIC T CELL RECEPTOR GENE**

(30) Priority: 18.07.2014 US 201462026348 P; 18.07.2014 US 201462026358 P
(71) Applicant: Kawamoto, Hiroshi, Kyoto-shi, Kyoto 606-0816 (JP)
(72) Inventor: KANEKO, Shin, Kyoto-shi Kyoto 606-8501 (JP); MASUDA, Kyoko, Kyoto-shi Kyoto 606-8501 (JP); MAEDA, Takuya, Kyoto-shi Kyoto 606-8501 (JP); NAGANO, Seiji, Kyoto-shi Kyoto 606-8501 (JP); KATSURA, Yoshimoto, Chiba 270-1166 (JP)
(74) Representative: Boult Wade Tennant
(86) International application number: PCT/JP2015/070624
(87) International publication number: WO 2016/010155

(57) **Abstract**

Provided is a method for inducing T cells for use in a cell-based immunotherapy, comprising the steps of:
(1) providing human pluripotent stem cells bearing a T cell receptor specific for a WT1 antigen or an Epstein-Barr virus associated antigen, and
(2) inducing T cell progenitors or mature T cells from the pluripotent stem cells provided in step (1). Pluripotent stem cells may preferably be iPS cells. The human pluripotent stem cells bearing a T cell receptor specific for an antigen may be prepared by inducing iPS cells from a T cell having the desired antigen specificity or by introducing genes encoding the T cell receptor specific for the desired antigen into iPS cells. The T cells obtained by this method can be used for the treatment of various immune-related diseases such as cancers and infectious diseases.

SEQUENCE LISTING

<110>  KAWAMOTO, Hiroshi

<120>  METHOD FOR PRODUCING PLURIPOTENT STEM CELL HAVING ANTIGEN
       SPECIFIC T CELL RECEPTOR GENE

<130>  672489

<150>  US62/026348
<151>  2014-07-18

<150>  US62/026358
<151>  2014-07-18

<160>  13

<170>  PatentIn version 3.5

<210>  1
<211>  9
<212>  PRT
<213>  Homo sapiens

<400>  1

Ile Tyr Val Leu Val Met Leu Val Leu
1               5

<210>  2
<211>  9
<212>  PRT
<213>  Homo sapiens

<400>  2

Cys Tyr Thr Trp Asn Gln Met Asn Leu
1               5

<210>  3
<211>  13
<212>  PRT
<213>  Homo sapiens

<400>  3

Cys Ile Val Thr Arg Phe Tyr Thr Asp Lys Leu Ile Phe
1               5                   10

<210>  4
<211>  16
<212>  PRT
<213>  Homo sapiens

<400>  4

Cys Ala Ser Ser Ser Pro Gly Ser Arg Pro Tyr Asn Glu Gln Phe Phe
1               5                   10                  15

<210>  5
<211>  14
<212>  PRT
<213>  Homo sapiens

<400>  5

Cys Ala Gly Glu Arg Gly Ser Thr Leu Gly Arg Leu Tyr Phe
1               5                   10


<210>  6
<211>  15
<212>  PRT
<213>  Homo sapiens

<400>  6

Cys Ala Ser Ser Pro Leu Ser Thr Gly Asn Tyr Glu Gln Tyr Phe
1               5                   10                  15


<210>  7
<211>  13
<212>  PRT
<213>  Homo sapiens

<400>  7

Cys Ala Met Ile Arg Gly Asn Thr Asp Lys Leu Ile Phe
1               5                   10


<210>  8
<211>  12
<212>  PRT
<213>  Homo sapiens

<400>  8

Cys Ala Ser Ser Phe Pro Ser Tyr Glu Gln Tyr Phe
1               5                   10


<210>  9
<211>  12
<212>  PRT
<213>  Homo sapiens

<400>  9

Cys Val Val Arg Gly Gly Gly Phe Lys Thr Ile Phe
1               5                   10


<210>  10
<211>  15
<212>  PRT
<213>  Homo sapiens

<400>  10

Cys Ser Ala Arg Ala Gly Thr Gly Gly Ala Asn Val Leu Thr Phe

1               5                   10                  15

<210>  11
<211>  28
<212>  DNA
<213>  Artificial Sequence

<220>
<223>  PCR Primer

<400>  11
cacaggctgt cttacaatct tgcagatc                                          28

<210>  12
<211>  23
<212>  DNA
<213>  Artificial Sequence

<220>
<223>  PCR primer

<400>  12
ctccacttcc agggctgcct tca                                               23

<210>  13
<211>  24
<212>  DNA
<213>  Artificial Sequence

<220>
<223>  PCR Primer

<400>  13
tgacctggga tggttttgga gcta                                              24

## Description

### ART RELATED

The present application relates to a method for generating pluripotent stem cells bearing genes encoding an antigen specific T cell receptor. Further, the present application relates to a method for generating T cells from thus obtained pluripotent stem cells. The present application also relates to a cell-based immunotherapy using the T cells induced from thus generated pluripotent stem cells.

### BACKGROUND ART

Wilms tumor 1(WT1) antigen is one of cancer antigens that have been intensively studied. WT1 is expressed at high frequency in various kinds of solid cancers. WT1 plays a role in maintenance of the malignant state of the cancer and is often observed in cancer stem cells. Clinical studies of WT1 vaccines for inducing WT1-antigen specific cytotoxic T Lymphocytes (CTL) have been underwent.

WT1 antigen specific T cell receptor (TCR) genes have been isolated. Clinical study of a gene therapy in which the TCR genes are forced to be expressed in normal T cells obtained from a patient and the TCR-expressing T cells are transplanted back to the patient has been conducted. Normal T cells derived from the patient are aggregates of various clones. In this study, TCRs inherently expressed in the normal T cells derived from the patient are suppressed by means of, for example, siRNA.

Epstein-Barr(EB) virus causes various diseases such as infectious mononucleosis, malignant lymphoma or Burkitt's lymphoma, and upper pharynx cancer. EB virus infected cells express virus derived proteins and those proteins can be targeted as antigens. That is, the cancer expresses foreign antigen and therefore, easy for targeting in an immunotherapy.

Latent membrane protein 2 (LMP2) is often utilized as a target antigen in a cell-based immunotherapy among EB virus associated antigens. LMP2 antigen specific T cell receptor (TCR) genes have been isolated. A gene therapy in which the TCR genes are forced to be expressed in normal T cells obtained from a patient and the TCR-expressing T cells are transplanted back to the patient has been proposed. Normal T cells derived from the patient are aggregates of various clones. In this study, TCRs inherently expressed in the T cells derived from the patient are suppressed by means of, for example, siRNA.

In those TCR gene therapies, there are at least three problems. 1) TCR genes are introduced with a retroviral vector. That is, this is a gene therapy and the TCR-introduced T cells could become cancerous. 2) The endogenous TCR genes may not be completely suppressed by siRNA. Dangerous T cell reaction could unintentionally be exerted through swapping between endogenous TCRa or TCRb chain and introduced TCRa or TCRb chain. 3) Collection of T-cells from the patient, gene modification and administration of the modified T cells to the patient must be conducted for every patient and therefore, previous preparation of the cells to be transplanted is difficult and the treatment takes time.

It has been proposed to collect WT1 specific CTLs or the like from the patient's peripheral blood and the cells are proliferated in the presence of a peptide to give cells for autologous transplantation. This procedure has been conducted with a significant efficiency by culturing the cells in the presence of IL-21 (Capuis et al, Science Translational Medicine, 5: 174ra27, 2013). This procedure, however, still have deficiencies of 4) Technical difficulty for producing a large amount of WT1 specific CTLs and 5) TCRs of the proliferated T cell clones are different in their affinity or specificity among the patients, and therefore, stable results are hardly achieved. In addition, prediction of the risk of cross-reaction in a given patient is very difficult.

### PRIOR ART DOCUMENTS

### PATENT DOCUMENTS

[Patent Literature 1] WO2011/096482

### NON PATENT DOCUMENT

[Non-Patent Literature 1] Chapuis et al, Sci Transl Med, 5:174ra27, 2013
[Non-Patent Literature 2] Vizcardo et al., Cell Stem Cell. 12: 31-36. 2013.
[Non-Patent Literature 3] Takahashi and Yamanaka, Cell 126, 663-673 (2006)
[Non-Patent Literature 4] Takahashi et al., Cell 131, 861-872(2007)
[Non-Patent Literature 5] Grskovic et al., Nat. Rev. Drug Dscov. 10,915-929(2011)
[Non-Patent Literature 6] Anticancer Research 32(12); 5201-5209, 2012
[Non-Patent Literature 7] Jurgens et al, Journal of Clinical Investigation, 26:22, 2006
[Non-Patent Literature 8] Morgan R.A. et al, Science, 314:126. 2006
[Non-Patent Literature 9] Timmermans et al., Journal of Immunology, 2009, 182: 6879-6888
[Non-Patent Literature 10] Blood 111:1318(2008)
[Non-Patent Literature 11] Nature Immunology 11: 585(2010)

### SUMMARY OF INVENTION

An object of the present application is to provide a method for producing pluripotent stem cells that bear genes encoding a T cell receptor specific for a given antigen, especially specific for a WT1 antigen or for an EB virus associated antigen. In another aspect, an object of the present application is to provide a method for generating T cells from thus prepared pluripotent stem cells. In a further aspect, an object of the present application is to provide a cell-based immunotherapy that uses the T cells generated from the pluripotent stem cells.

In a further aspect, an object of the present application is to provide pluripotent stem cells bearing genes encoding a T cell receptor specific for an antigen, especially, for a WT1 antigen or an EB virus associated antigen.

In the first embodiment of the present application, a method for generating pluripotent stem cells comprising the steps of isolating or inducing WT1 antigen specific T cells or EB virus associated antigen specific T cells from a donor and inducing pluripotent stem cells from the T cells. In the case where the pluripotent stem cells are iPS cells, the iPS cells established from a T cell are represented as "T-iPS cells". The donor of the WT1 antigen specific T cells may be a healthy person or a WT1 antigen bearing cancer patient. The donor of EB virus associated antigen specific T cells may be a healthy person, a patient suffered from an EB virus associated disease or a person previously infected with EB virus but did not develop any EB virus associated disease.

In the 2nd embodiment, the present application provides a method for generating pluripotent stem cells that bear genes encoding a T cell receptor specific for a WT1 antigen or an EB virus associated antigen. In this specification, iPS cells obtained by introducing the TCR genes are called as TCR-iPS cells. When TCR-iPS cells are used as source for a cell-based immunotherapy, the method is called as TCR-iPS cell therapy.

The present application further provides a method for generating T cells to be used for the cell-based immunotherapy, which comprises generating mature T cells or T progenitor cells from the pluripotent stem cells bearing genes encoding a T cell receptor specific for a WT1 antigen or an EB virus associated antigen.

T cell progenitors or mature T cells obtained by the method of the present application may be used for the cell-based immunotherapy. In the cell-based immunotherapy, the mature T cells obtained by this invention may be used for autograft and also for allograft to whom having HLAs that match with the donor's HLA to a predetermined extend.

An idea of transplanting cells derived from another person does not meet the common technical knowledge regarding conventional a cell-based immunotherapy. For example, bone marrow transplantation that transplants hematopoietic stem cells has been employed for treating malignant hematological cancers such as leukemia. In this therapy, HLA must match between the donor and recipient so that the bone marrow from the donor will not be rejected by the recipient. However, amino acid sequences of various protein molecules other than HLAs in the body are different between the two individuals. T cells from the donor could recognize the recipient as target to be attacked due to those mismatches in the recipient. As a result, a part of the transplanted T cells derived from the donor may fight against the recipient's cells, i.e. graft-versus-host disease (GVHD) could occur. When HLAs between donor and recipient do not match, the graft-versus-host disease will be more severe. In fact, GHVDs have been observed frequently in patients who receive bone marrow transplantation. The art in the medical field recognizes that the transplantation of allogenic T cells is highly risky.

By using T cells induced from pluripotent stem cells bearing genes encoding a T cell receptor specific for a desired antigen, the inventors could unexpectedly solve the above recognized problems to some extent.

The T-iPS cell therapy of the first embodiment does not a gene therapy and therefore, there is no risk of oncogenesis that could be occurred by introducing genes into the cells. CTLs re-generated from the iPS cells express only the introduced TCR genes while re-arrangement of the endogenous TCR genes are suppressed and therefore, unexpected attack by the regenerated T cells may not occur. For more safe therapy, expression of endogenous TCR genes in the iPS cells may be suppressed. TCR-iPS cells may be obtained as clonally expanded cells. A safe clone may be determined by identifying the site to which the genes are introduced and used to avoid injuring the recipient's genes.

In the present application, pluripotent stem cells bearing genes encoding a T cell receptor specific for a given antigen are generated, and T cell progenitors or mature T cells are re-generated from said pluripotent stem cells. Thus generated T cell progenitors or mature T cells are used for the cell-based immunotherapy. The cells to be transplanted to the patient will be T cells having single antigen specificity and therefore, will not cause GVHD. Accordingly, the method of the present application may be used not only for autologous but also for allogenic transplantation. According to the present application, allogenic transplantation of T cells has become possible for the first time.

In various proposed therapies wherein various cells or tissues, other than T cells, that are differentiated from iPS cells are transplanted, the cells to be transplanted cells are expected to be fixed in the body of the patient for his/her entire life. In regenerative therapies that use cells or tissues regenerated from iPS cell stock for allogenic transplantation, the patients need to take immune suppressing drugs for their entire life. This is disadvantageous point compared to autologous transplantation. On the other hand, T cells regenerated from T-iPS or TCR-iPS cells according to the present application, the allogenically transplanted T cells are eventually rejected after a certain period. That is, allogenic graft will be eventually rejected based on mismatches of minor histocompatibility antigens even in the HLA-matched donor and recipient. In this point, the cell-based immunotherapy provided by this application is advantageous than the other allogenic transplantation of the cells or tissues regenerated from iPS cells.

Various types of T-iPS cells or TCR-iPS cells may be produced in advance. There is no need for producing T-iPS cells or TCR-iPS cells for each patient. A bank of T-iPS cells or TCR-iPS cells, or a bank of T cell progenitors or mature T cells produced from those iPS cells may be established. The present application accelerates the establishment of the therapy in which allogenic T cells are transplanted. This method has advantages not only of shortening the period for preparation of the cell-based immunotherapy but also enabling the verification of the quality of the cells before transplantation.

### BRIEF DESCRIPTION OF DRAWINGS

Figure 1 is a result of FACS analysis of the cells
Figure 11 is result of FACS analysis showing that WT1 tetramer positive-CD8 positive T cells were induced from T cells derived from a healthy volunteer in example 3.
Figure 12 is a photograph of an iPS cell colony derived from a WT1 peptide specific T cell.

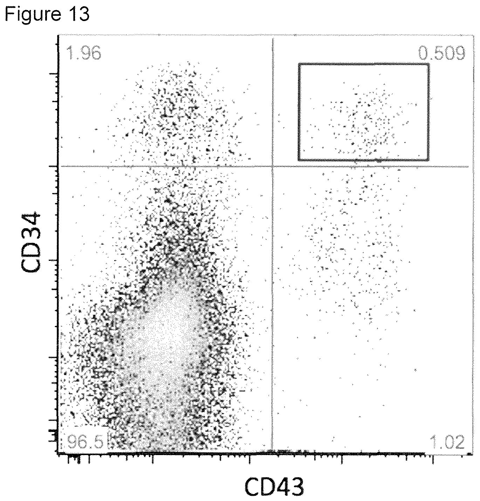

Figure 13 is a result of FACS analysis on day 13 of the differentiation of T-iPS cells established from a WT1 peptide specific T cell into T cells.
Figure 14 is a result of FACS analysis on day 36 of the differentiation of T-iPS cells established from a WT1 peptide specific T cell into T cells.
Figure 15 shows peptide specific cytotoxicity of the CTLs regenerated from clone WT1#9 established in Example 3 against LCLs.
Figure 16 shows peptide specific cytotoxicity of the CTLs regenerated from clone WT1#3-3 established in Example 4 against LCLs.
Figure 17 shows cytotoxic activity of the CTLs regenerated from clone WT1#3-3 against THP1 leukemia cells. The cytotoxic activity of the cells was completely blocked by an anti-HLA class I antibody.
Figure 18 shows cytotoxic activity of the CTLs regenerated from clone WT1#3-3 against HL60 leukemia cells. The cytotoxic activity of the cells was completely blocked by an anti-HLA class I antibody.
Figure 19 shows pTA vector.
Figure 20 shows CS-UbC-RfA-IRES2-Venus vector.
Figure 21 shows WT1 specific TCR genes were duly introduced in the iPS cells.

### DETAILED DESCRIPTION OF PREFERRED EMBODIMENTS

obtained in Example 1. LMP2 tetramer positive-CD8 positive T cells were induced from T cells derived from a healthy volunteer.

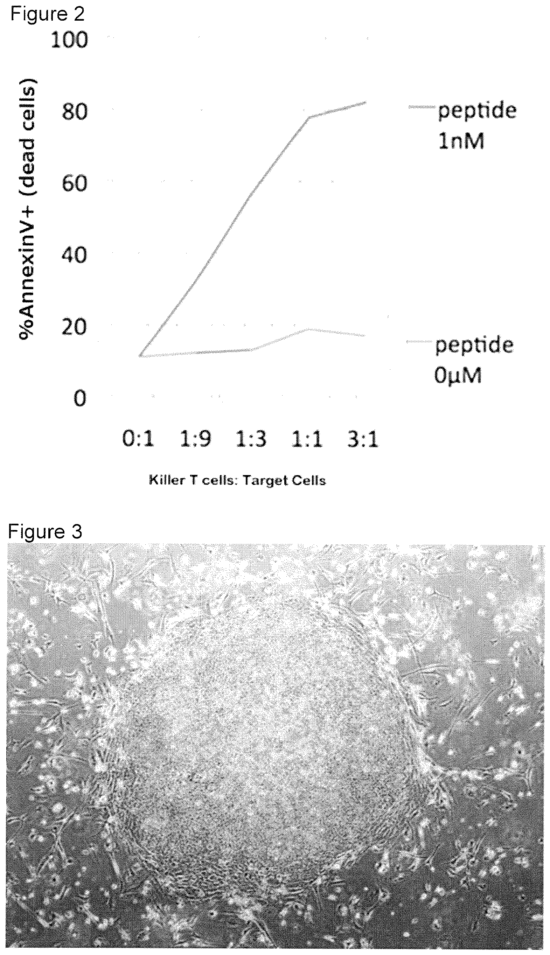

Figure 2 shows that T cells induced by using LMP2 peptide from peripheral blood obtained from a healthy volunteer having HLA-A2402 who had previously been infected with EB virus exerted peptide specific killer activity.

Figure 3 is a photograph of an iPS cell colony induced from a LMP2 peptide specific T cell.

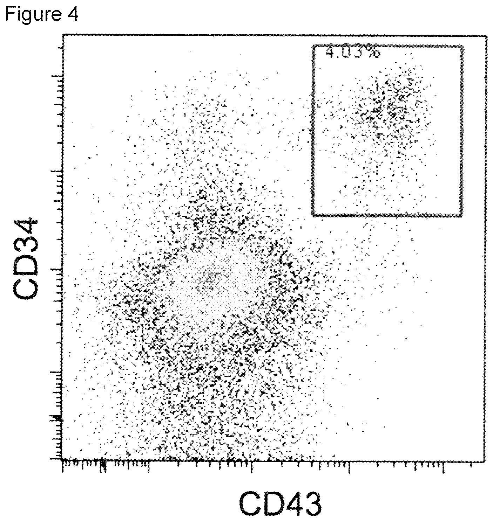

Figure 4 is a result of FACS analysis on day 13 of the differentiation of T-iPS cells established from LMP2 peptide specific T cells into T cells.

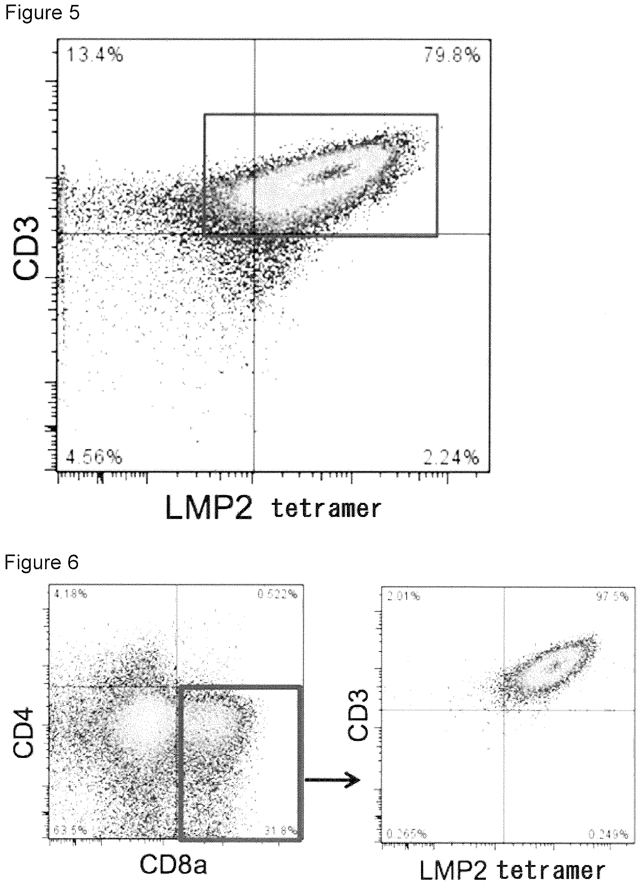

Figure 5 is a result of FACS analysis on day 36 of the differentiation of T-iPS cells established from LMP2 peptide specific T cells into T cells.

Figure 6 is a result of FACS analysis on day 41 of the differentiation of T-iPS cells established from LMP2 peptide specific T cells into T cells. Generation of LMP2 specific mature T cells (CTLs) was confirmed.

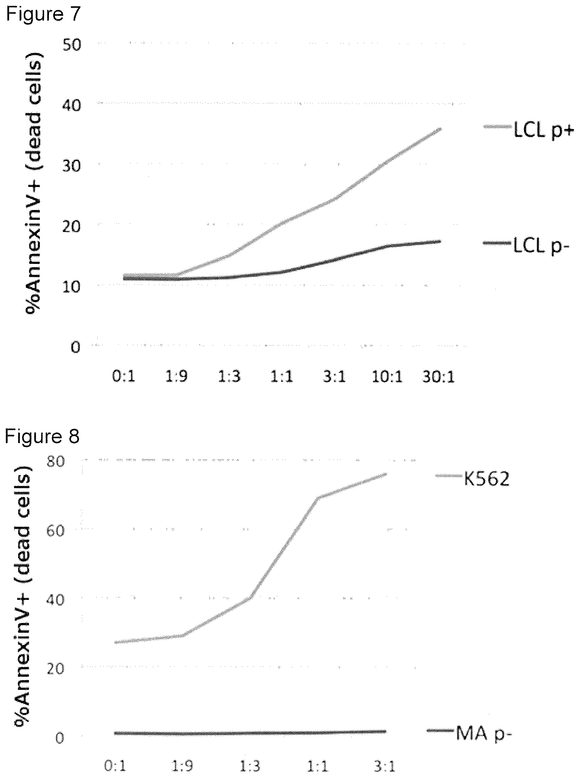

Figure 7 shows LMP2 specific killer activity of the mature T cells (CTLs) regenerated from T-iPS cells established from a LMP2 peptide specific T cell. The killer activities in the presence (p+) or absence (p-) of LMP2 peptide were observed by using LCLs as target cells.

Figure 8 shows natural killer cell-like activities of mature T cells regenerated from T-iPS cells established from a LMP2 peptide specific T cell.

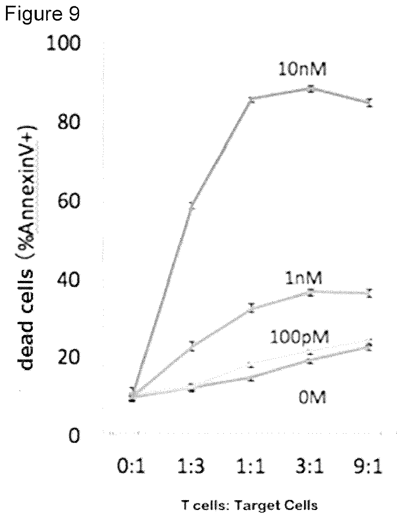

Figure 9 shows peptide specific cytotoxicity of CTLs regenerated from clone LMP2#1 against LCLs.

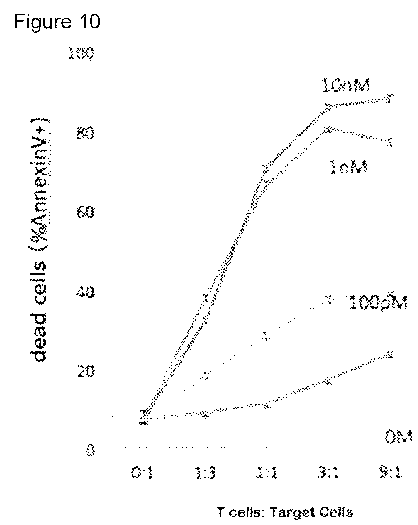

Figure 10 shows peptide specific cytotoxicity of CTLs regenerated from clone LMP2#13 obtained in Example 2 against LCLs.

In the specification and claims, "pluripotent stem cells" refer to stem cells having pluripotency, i.e. an ability to differentiate into many types of cells in the body, and self-propagation ability. Examples of pluripotent stem cells may include embryonic stem cells (ES cells), nuclear transfer embryonic stem cells (ntES cells), embryonic germ cells (EG cells), and induced pluripotent stem cells (iPS cells). According to the present application, pluripotent stem cells are preferably those derived from a mammal and especially from a human.

In the specification and claims, "T cells" refer to cells expressing receptors for antigens called as T cell receptor (TCR). The fact that TCR of a T cell is maintained in iPS cells established from the T cell has been reported by WO2011/096482 and Vizcardo et al., Cell Stem Cell 12, 31-36 2013.

In the 1st embodiment of the present application, iPS cells are induced from a T cell having a desired antigen specificity. T cells used as origin for iPS cells may preferably be T cells expressing at least one of CD4 and CD8, in addition to CD3. Examples of the preferable human T cells my include helper/regulatory T cells that are CD4 positive cells; cytotoxic T cells that are CD8 positive cells; naive T cells that are CD45RA⁺CD62L⁺ cells; central memory T cells that are CD45RA⁻CD62L⁺ cells, effector memory T cells that are CD45RA⁻CD62L⁻ cells and terminal effector T cells that are CD45RA⁺CD62L⁻ cells.

Human T cells can be isolated from a human tissue by known procedures. The human tissue is not limited in particular, if the tissue contains T cells of the above-mentioned type, and examples thereof include peripheral blood, lymph node, bone marrow, thymus, spleen, umbilical cord blood, and a lesion site tissue. Among these, peripheral blood and umbilical cord blood are preferable since they can be derived less invasively from the human body and can be prepared with ease. Known procedures for isolating human T cells include, for example, flow cytometry using an antibody directing to a cell surface marker, such as CD4, and a cell sorter, as shown in the below-mentioned Examples. Alternatively, desired T cells can be isolated by detecting the secretion of a cytokine or the expression of a functional molecule as an indicator. In this case, for example, T cells secrete different cytokines, depending on whether they are of the Th1 or Th2 type, and thus T cells of a desired Th type can be isolated by selecting T cells using the cytokine as an indicator. Similarly, cytotoxic (killer) T cells can be isolated using the secretion or production of granzyme, perforin, or the like as an indicator.

WT1 antigen specific cytotoxic T cells may be obtained by stimulating lymphocyte obtained from a human by a conventional procedure with WT1 or an epitope peptide thereof. Various WT1 antigen epitope peptides have been identified and the procedures for inducing WT1 specific cytotoxic T cells with those peptides have been well known. Alternatively, lymphocytes may be stimulated by cancer cells expressing a WT1 antigen.

WT1 antigen specific cytotoxic T cells may be induced from the cells obtained from an individual who is suffered from cancer that expresses WT1 or an individual who had previously been suffered from cancer that expresses WT1. WT1 antigen specific cytotoxic T cells may also be induced from a healthy volunteer.

EB virus associated antigen specific cytotoxic T cells may be induced by stimulating lymphocyte obtained from a human by a conventional procedure with an EB virus associated antigen, such as LMP2 antigen or an epitope peptide thereof. Various EB virus associated antigen epitope peptides have been identified and the procedures for inducing EB virus specific cytotoxic T cells with those peptides have been well known. Alternatively, lymphocytes may be stimulated by cancer cells expressing EB virus associated antigen.

EB virus associated antigen specific cytotoxic T cells may be induced from cells of a patient suffered from an EB virus infectious disease or an EB virus associating cancer, a healthy individual who is a carrier of EB virus or a healthy individual who have never been infected with EB virus.

In general, antigen specific T cells may be obtained from a patient with the infectious disease or cancer. This is because antigen specific T cells are proliferated in the body of the patient and therefore, it may be easy to detect/obtain T cells with specific reactivity. According to the present application, T-iPS cells for allogenic transplantation may be induced from a T cell having specificity for a disease obtained from a patient having the disease. In the present application, the antigen specific T cells may also be obtained from a healthy volunteer. By using T cells obtained from a healthy volunteer, the following advantages can be achieved: 1) Various antigen specific T cells may be induced from a healthy volunteer's cells and therefore, a variety of T-iPS cells having various TCR genes can be prepared in advance. 2) It is easier to collect donors from healthy people for establishing a T-iPS cell bank.

Human T cells specific for a given antigen may be isolated from cell culture or tissue containing WT1 antigen specific or EB virus associated antigen specific T cells with an affinity column immobilized with the desired antigen. Alternatively, tetramer of an antigen-bound major histocompatibility complex (MHC) may be used to isolate human T cells specific for a given antigen, such as a WT1 antigen or an EB virus associated antigen from human tissues.

iPS cells may be induced from thus obtained T cell specific for a WT1 antigen or an EB virus associated antigen. The procedure for inducing pluripotent stem cells from T cells may be those taught by Vizcardo et al. , Cell Stem Cell 12, 31-36 2013. For example, T cells specific for a given antigen may be obtained from an individual who had acquired immunity against the disease to be treated and the Yamanaka factors may be introduced to the T cells to give iPS cells (Takahashi and Yamanaka, Cell 126, 663-673 (2006), Takahashi et al., Cell 131, 861-872(2007) and Grskovic et al., Nat. Rev. Drug Dscov. 10,915-929 (2011).

Induced pluripotent stem (iPS) cells can be prepared by introducing specific reprogramming factors to somatic cells. iPS cells are somatic cell-derived artificial stem cells having properties almost equivalent to those of ES cells (K. Takahashi and S. Yamanaka(2006) Cell, 126:663-676; K. Takahashi et al. (2007), Cell, 131 :861-872; J. Yu et al. (2007), Science, 318:1917-1920; Nakagawa, M. et al., Nat. Biotechnol. 26:101-106(2008); and WO 2007/069666). The reprogramming factors may be constituted by genes or gene products thereof, or non-coding RNAs, which are expressed specifically in ES cells; or genes or gene products thereof, non-coding RNAs or low molecular weight compounds, which play important roles in maintenance of the undifferentiated state of ES cells. Examples of genes included in the reprogramming factors include Oct3/4, Sox2, Sox1, Sox3, Sox15, Sox17, Klf4, Klf2, c-Myc, N-Myc, L-Myc, Nanog, Lin28, Fbxl5, ERas, ECAT15-2, Tell, beta-catenin, Lin28b, Sall1, Sall4, Esrrb, Nr5a2, Tbx3 and Glis1, and these reprogramming factors may be used either individually or in combination. Examples of the combination of the reprogramming factors include those described in WO2007/069666; WO2008/1 18820; WO2009/007852; WO2009/032194; WO2009/058413; WO2009/057831; WO2009/075119; WO2009/079007; WO2009/091659; WO2009/101084; WO2009/101407; WO2009/102983; WO2009/1 14949; WO2009/1 17439; WO2009/126250; WO2009/126251; WO2009/126655; WO2009/157593; WO2010/009015; WO2010/033906; WO2010/033920; WO2010/042800; WO2010/050626; WO 2010/056831; WO2010/068955; WO2010/098419; WO2010/102267; WO 2010/11 1409; WO 2010/1 11422; WO2010/1 15050; WO2010/124290; WO2010/147395; WO2010/147612; Huangfu D, et al. (2008), Nat. Biotechnol., 26: 795-797; Shi Y, et al. (2008), Cell Stem Cell, 2: 525-528; Eminli S, et al. (2008), Stem Cells. 26:2467-2474; Huangfu D, et al. (2008), Nat Biotechnol. 26: 1269-1275; Shi Y, et al. (2008), Cell Stem Cell, 3, 568-574; Zhao Y, et al. (2008), Cell Stem Cell, 3:475-479; Marson A, (2008), Cell Stem Cell, 3, 132-135; Feng B, et al. (2009), Nat Cell Biol. 1 1 :197-203; R.L. Judson et al.(2009),Nat. Biotech., 27:459-461 ; Lyssiotis CA, et al. (2009), Proc Natl Acad Sci U S A. 106:8912-8917; Kim JB, et al. (2009), Nature. 461 :649-643; Ichida JK, et al. (2009), Cell Stem Cell. 5:491-503; Heng JC, et al. (2010), Cell Stem Cell. 6: 167-74; Han J, et al. (2010), Nature. 463:1096-100; Mali P, et al. (2010), Stem Cells. 28:713-720, and Maekawa M, et al. (2011), Nature. 474:225-9. The contents of the documents cited in this paragraph are herein incorporated by reference.

The reprogramming factors may be contacted with or introduced into the somatic cells by a known procedure suitable for the form of the factor to be used.

In the case where the reprogramming factors are in the form of protein, the reprogramming factors may be introduced into somatic cells by a method such as lipofection, fusion with a cell-permeable peptide (e.g., HIV-derived TAT or polyarginine), or microinjection.

In the case where the reprogramming factors are in the form of DNA, the reprogramming factors may be introduced into somatic cells by a method such as use of a vector including virus, plasmid and artificial chromosome vectors; lipofection; use of liposome; or microinjection. Examples of the virus vector include retrovirus vectors, lentivirus vectors (these are described in Cell, 126, pp. 663-676, 2006; Cell, 131, pp. 861-872, 2007; and Science, 318, pp. 1917-1920, 2007), adenovirus vectors (Science, 322, 945-949, 2008), adeno-associated virus vectors and Sendai virus vectors (WO 2010/008054). Examples of the artificial chromosome vector include human artificial chromosome (HAC), yeast artificial chromosome (YAC), and bacterial artificial chromosome (BAC and PAC). Examples of the plasmid which may be used include plasmids for mammalian cells (Science, 322:949-953, 2008). The vector may contain a regulatory sequence(s) such as a promoter, enhancer, ribosome binding sequence, terminator and/or polyadenylation site to enable expression of the nuclear reprogramming factors; and, as required, a sequence of a selection marker such as a drug resistance gene (e.g., kanamycin-resistant gene, ampicillin-resistant gene or puromycin-resistant gene), thymidine kinase gene or diphtheria toxin gene; a gene sequence of a reporter such as the green-fluorescent protein (GFP), β-glucuronidase (GUS) or FLAG. Further, in order to remove, after introduction of the gene into the somatic cells and expression of the same, the genes encoding the reprogramming factors, or both the promoter(s) and the genes encoding the reprogramming factors linked thereto, the vector may have LoxP sequences upstream and downstream of these sequences.

Further, in the case where the reprogramming factors are in the form of RNA, each reprogramming factor may be introduced into somatic cells by a method such as lipofection or microinjection, and an RNA into which 5-methylcytidine and pseudouridine (TriLink Biotechnologies) were incorporated may be used in order to suppress degradation (Warren L, (2010) Cell Stem Cell. 7:618-630). The documents cited in this paragraph are herein incorporated by reference.

Examples of the medium for inducing iPS cells include DMEM, DMEM/F12 and DME media supplemented with 10 to 15% FBS (these media may further contain LIF, penicillin/streptomycin, puromycin, L-glutamine, non-essential amino acids, β-mercaptoethanol and/or the like, as appropriate); and commercially available media [for example, medium for culturing mouse ES cells (TX-WES medium, Thromb-X), medium for culturing primate ES cells (medium for primate ES/iPS cells, ReproCELL) and serum-free medium (mTeSR, Stemcell Technology)].

Examples of the method to induce iPS cells include a method wherein somatic cells and reprogramming factors are brought into contact with each other at 37°C in the presence of 5% CO₂ on DMEM or DMEM/F12 medium supplemented with 10% FBS, and the cells are cultured for about 4 to 7 days, followed by plating the cells on feeder cells (e.g., mitomycin C-treated STO cells or SNL cells) and starting culture in a bFGF-containing medium for culturing primate ES cells about 10 days after the contact between the somatic cells and the reprogramming factors, thereby allowing ES-like colonies to appear about 30 to about 45 days after the contact, or later.

Alternatively, the cells may be contacted with the reprogramming factors and cultured at 37°C in the presence of 5% CO₂ on feeder cells (e.g., mitomycin C-treated STO cells or SNL cells) in DMEM medium supplemented with 10% FBS (this medium may further contain LIF, penicillin/streptomycin, puromycin, L-glutamine, non-essential amino acids, β-mercaptoethanol and the like, as appropriate) for about 25 to about 30 days or longer, thereby allowing ES-like colonies to appear. Preferred examples of the culture method include a method wherein the somatic cells themselves to be reprogrammed are used instead of the feeder cells (Takahashi K, et al. (2009), PLoS One. 4:e8067 or WO2010/137746), and a method wherein an extracellular matrix (e.g., Laminin-5 (WO2009/123349), Laminin-5 (WO2009/123349), Laminin-10 (US2008/0213885) or its fragment (WO2011/043405) or Matrigel (BD)) is used instead. The documents cited in this paragraph are herein incorporated by reference.

Other examples include a method wherein the iPS cells are established using a serum-free medium (Sun N, et al. (2009), Proc Natl Acad Sci U S A. 106: 15720-15725). Further, in order to enhance the establishment efficiency, iPS cells may be established under low oxygen conditions (at an oxygen concentration of 0.1 % to 15%) (Yoshida Y, et al. (2009), Cell Stem Cell. 5:237-241 or WO2010/013845). The contents of the documents cited in this paragraph are herein incorporated by reference.

Examples of factors used for enhancing the establishment efficiency may include histone deacetylase (HDAC) inhibitors [e.g., low-molecular inhibitors such as valproic acid (VPA), trichostatin A, sodium butyrate, MC 1293, and M344, nucleic acid-based expression inhibitors such as siRNAs and shRNAs against HDAC (e.g., HDAC1 siRNA Smartpool.RTM. (Millipore), HuSH 29mer shRNA Constructs against HDAC1 (OriGene) and the like), and the like], MEK inhibitor (e.g., PD184352, PD98059, U0126, SL327 and PD0325901), Glycogen synthase kinase-3 inhibitor (e.g., Bio and CHIR99021), DNA methyl transferase inhibitors (e.g., 5-azacytidine), histone methyl transferase inhibitors [for example, low-molecular inhibitors such as BIX-01294, and nucleic acid-based expression inhibitors such as siRNAs and shRNAs against Suv39h1, Suv39h2, SetDB1 and G9a], L-channel calcium agonist (for example, Bayk8644), butyric acid, TGFβ inhibitor or ALK5 inhibitor (e.g., LY364947, SB431542, 616453 and A-83-01), p53 inhibitor (for example, siRNA and shRNA against p53), ARID3A inhibitor (e.g., siRNA and shRNA against ARID3A), miRNA such as miR-291-3p, miR-294, miR-295, mir-302 and the like, Wnt Signaling (for example, soluble Wnt3a), neuropeptide Y, prostaglandins (e.g., prostaglandin E2 and prostaglandin J2), hTERT, SV40LT, UTF1, IRX6, GLIS1, PITX2, DMRTB1 and the like. Upon establishing iPS cells, a medium added with the factor for enhancing the establishment efficiency may be used.

During the culture, the medium is replaced with the fresh medium once every day from Day 2 of the culture. The number of somatic cells used for nuclear reprogramming is not restricted, and usually within the range of about 5×10³ to about 5×10⁶ cells per 100 cm² area on the culture plate.

iPS cells may be selected based on the shape of each formed colony. In cases where a drug resistance gene is introduced as a marker gene such that the drug resistance gene is expressed in conjunction with a gene that is expressed when a somatic cell was reprogrammed (e.g., Oct3/4 or Nanog), the established iPS cells can be selected by culturing the cells in a medium containing the corresponding drug (selection medium). Further, iPS cells can be selected by observation under a fluorescence microscope in cases where the marker gene is the gene of a fluorescent protein. Thus induced iPS cells (T-iPS cells) bear genes encoding the T cell receptor derived from the original T cell from which the iPS cells were induced.

In the 2nd embodiment according to this application, TCR genes specific for a WT1 antigen or an EB virus associated antigen are introduced into pluripotent stem cells.

TCRs specific for a WT1 antigen and those for an EB virus associated antigen in relation to various cancers have been reported. For example, see Anticancer Research 32(12); 5201-5209, 2012 and Jurgens et al, Journal of Clinical Investigation, 26:22, 2006. TCR genes may be obtained from T cells specific for a given antigen isolated from a patient having a cancer or an infectious disease. The TCR genes may be isolated from thus obtained T cells. In this application, TCR genes specific for a given antigen may be introduced into pluripotent stem cells such as iPS cells that were induced from the donor cells. For example, this procedure may be conducted as taught by Morgan R.A. et al, Science, 314:126. 2006. In particular, a suitable vector bearing the TCR genes may be introduced into the iPS cells. For example, TCR genes may be introduced by a vector such as virus, plasmid and artificial chromosome vectors; or by means of lipofection, liposome or microinjection. Examples of the virus vectors include retrovirus vectors, lentivirus vectors, adenovirus vectors, adeno-associated virus vectors and Sendai virus vectors. Examples of the artificial chromosome vector include human artificial chromosome (HAC), yeast artificial chromosome (YAC), and bacterial artificial chromosome (BAC and PAC). Examples of the plasmid which may be used include plasmids for mammalian cells. The vector may contain a regulatory sequence(s) such as a promoter, enhancer, ribosome binding sequence, terminator and/or polyadenylation site to enable expression of the TCR genes. If desired, the vector may also contain a selection marker such as a drug resistance gene (e.g., kanamycin-resistant gene, ampicillin-resistant gene or puromycin-resistant gene), thymidine kinase gene or diphtheria toxin gene; and a reporter such as the green-fluorescent protein (GFP), β-glucuronidase (GUS) or FLAG.

The pluripotent stem cells expressing the desired TCR genes are then differentiated into T cell progenitors or mature T cells. The procedure for differentiating pluripotent stem cells into T cells may be that taught by Timmermans et al., Journal of Immunology, 2009, 182: 6879-6888.

In the specification and claims, "T cell progenitors" may cover cells at any stages of the T cell development, from undifferentiated cells corresponding to hematopoietic stem cells to the cells at the stage just before the cells undergo positive selection/negative selection. Details of the differentiation of T cells are explained in Blood 111:1318(2008) and Nature Immunology 11: 585(2010).

T cells are roughly divided into αβ T cells and γδ T cells. αβ T cells include killer T cells and helper T cells. In this application "T cells" cover all types of T cells. T cells may cover any of T progenitor cells and mature T cells. Preferably, T cells may be those expressing at least one of CD4 and CD8 in addition to CD3.

A significantly high proportion of hematological malignancies and solid tumors express WT1 antigen. The method of the present application can be used in the cell-based immunotherapy for WT1 expressing cancers.

According to the present application, T cell preparations targeting for a WT1 cancer antigen or an EB virus associated antigen, such as LMP2 are provided. In one embodiment, T-iPS cells may be induced from WT1 antigen specific T cells or LMP2 antigen specific T cells obtained from a normal person and the T-iPS cells may be differentiated or re-generated into T cells. Function of the T cells re-generated from T-iPS cells may be verified with the cells obtained from the person from whom the T-iPS cells were induced and then, the T-iPS cells are stored to create a cell bank.

The HLA of the patient with a WT1 antigen-expressing cancer may be determined and HLA-matched T-iPS cells may be chosen from the T-iPS cell bank. Then, T cells re-generated from the T-iPS cells may be used for the cell-based immunotherapy. Alternatively, T-iPS cells may be differentiated into T cells and then the T cells are frozen and stored. By the latter procedure, a cell-based immunotherapy for more patients can be started more quickly. In addition, the transferred cells are rejected eventually and therefore, there is no need to consider the risk of oncogenic transformation of the transferred cells.

In the cell-based immunotherapy method of the present application, the re-generated T cells are dispersed in a suitable media such as saline or PBS and the dispersion may be administered to a patient having a certain matching level of the HLA to the donor. The matching level of the donor and the patient may be complete match. When the donor is homozygous for HLA haplotype (hereinafter referred to as "HLA haplotype homo") and the patient is heterozygous for HLA haplotypes (hereinafter referred to as "HLA haplotype hetero"), one of the patient's HLA haplotypes should match the donor's homozygous HLA haplotype. The cells may be administered intravenously. The amount of the cells to be administered is not limited. Cells that have been differentiated into mature T cells may be intravenously administered once to several times in an amount of 10⁶-10⁷ cells/kg per administration. T cell progenitors may be administered in an amount of about 1/10-1/100 of that of the mature T cells.

The number of the cells to be administered is not limited and may be determined based on, for example, the age, sex, height and body weight of the patient and disease and conditions to be treated. The optimal cell number may be determined through clinical studies.

T cells may target various antigens and therefore, the method of this application may be applied for a cell-based immunotherapy against various diseases including cancers, infectious diseases, autoimmune diseases and allergies.

A high proportion of hematopoietic organ tumors such as leukemia, myelodysplastic syndrome, multiple myeloma, and malignant lymphoma, as well as solid tumors such as stomach cancer, colon cancer, lung cancer, breast cancer, germ cell cancer, liver cancer, skin cancer, bladder cancer, prostate cancer, uterine cancer, cervical cancer and ovarian cancer express the WT1 gene. Accordingly, CTL cells generated from WT1 specific T-iPS cells or TCR-iPS cells are effective for the cell-based immunotherapy for those WT1 gene expressing cancers.

Epstein-Barr (EB) virus causes various diseases such as infectious mononucleosis as well as cancers such as malignant lymphoma or burkitt lymphoma and epipharyngeal carcinoma. CTL cells differentiated from T-iPS cells or TCR-iPS cells having TCR genes specific for LMP2 antigen, an EB virus associated antigen, may be useful for the treatment of EB virus associated infectious diseases or cancers, for example, Hodgkin's disease, barkitt lymphoma, nasopharyngeal carcinoma, some types of stomach cancer, and a cell-based immunotherapy for transplantation.

### [EXAMPLE 1]

T-iPS cells (clone LMP2#1) were established from a T cell specific for LMP2 antigen derived from peripheral blood mononuclear cells of an EB virus carrier. T-iPS cells were differentiated into LMP2 antigen specific CTLs (herein after, referred to as "re-generated LMP2-CTL#1").

EB virus infection in acute phase may cause infectious mononucleosis and sometimes cause cancer such as barkitt lymphoma. In this example, the donor for T cells was a healthy person who had previously been infected with EB virus. Once infected, this virus stays in the lymphocytes for entire life and therefore, the donor is an EB virus carrier. The donor is, therefore, considered to have chronic EB virus infection.

### 1) Propagation of cytotoxic T Lymphocytes (CTL) specific for LMP2 antigen

i) The following media were used.
Medium for dendritic cells: CellGro (CellGenix)

**[Table 1]**

| Medium for T cells (T cell medium): | | |
|---|---|---|
| | Amount | Final conc. |
| RPMI | 45ml | |
| human AB serum | 5ml | 10% |
| Total | 50ml | |

ii) The LMP2 antigen peptide used is as follows.
LMP2: IYVLVMLVL (SEQ ID NO: 1)
LMP2 tetramer was purchased from MBL.

iii) The LCL (Lymphoblastoid cell line) used is as follows.

Lymphoblastoid cell line (LCL) established from healthy volunteer A who had previously infected with EB virus and had HLA-A*02:06/24:02; B*39:01/40:02; C*07:02/15:02; DRB1*04:10/09:01 in the Department of Hematology and Oncology, Graduate School of Medicine, Kyoto University, Kyoto, Japan was used as antigen presenting cells.

### A. Induction of human monocyte dendritic cells (MoDC) from human peripheral blood

1. Peripheral blood was obtained from healthy volunteer A having HLA-A2402 who had previously been infected with EB virus. Monocytes were isolated from the blood by using CD14 microbeads. The cells were washed and added with the medium for dendritic cells to give a 5×10⁵ cells/mL suspension.
2. Cytokines were added to the cell suspension to give final concentrations of GM-CSF 800 U/mL (or 50 ng/mL), IL-4 200 U/mL (or 40 ng/mL). Five milliliter (5mL) of the cell suspension was seeded to each well of a 6-well plate. The plate was incubated at 37°C with 5% CO₂.
3. The plate was incubated for 3 days and on day 3, 2.5mL of the culture supernatant was gently removed. Fresh medium for dendritic cells were added with GM-CSF and IL-4 to give final concentrations of 800 U/mL and 200 U/mL respectively.
4. Thus prepared fresh medium for dendritic cells 3mL was added to each well.
5. On day 6, immature monocyte-derived dendritic cells (MoDCs) were collected from the plate and added in a small amount of fresh medium for dendritic cells.
6. The density of the cell suspension was adjusted to 5×10⁵ cells/mL.
7. GM-CSF (final concentration: 800 U/mL), IL-4 (final concentration: 200 U/mL), TNF-alpha (final concentration: 10 ng/mL), and PGE2 (final concentration: 1 µg/mL) were added to the cell suspension. About 5×10⁵ cells/mL/well of the cell suspension was added to each well of a 24-well plate.
8. The plate was incubated at 37°C with 5% CO2 for 24 hours.
9. The peptide was added to each well in last 2 hours of the 24 hours incubation period. The final concentration of the peptide was 10 µM. Dendritic cells (DC) were collected from the plate and washed twice with the medium for T cells.
10. The number of the DCs was counted and the medium for T cells was added to give a 2×10⁵ cells/mL suspension.

### B. Isolation of T cells from human peripheral blood and co-culture of the T cells and dendritic cells.

1. T cells were isolated from peripheral blood of the healthy volunteer A (the same person in the step A above) by means of the MACS technique using CD3 microbeads. The cells were washed and added with the medium for T cells to give a 2×10⁶ cells/mL suspension. A small part of the T cell suspension was separated for the flow cytometry analysis.
2. 0.5 mL/well of DC cell suspension (2×10⁵ cells/mL) and 0.5 mL/well of the T cell suspension (2×10⁶ cells/mL) were added to each well of a 24 well plate. (DC cells: T cells=1×10⁵: 1×10⁶=1:10).
3. On day 3, IL-7 (final concentration: 5 ng/mL) and IL-15(final concentration: 10 ng/mL) were added to each well.
4. On day 14, the cells were collected from the culture.

### C. Addition of the peptide to LCL

1. LCLs were collected from the culture and irradiated at a dose of 35Gy.
2. The irradiated cells were suspended in the T cell medium to give a 5×10⁵ cells/mL suspension.
3. The peptide was added to the suspension 100nM and incubated for 2 hours.
4. The LCL were collected and washed with the T cell medium and then, dispersed in the T cell medium to give a 2×10⁵ cells/mL suspension.

### D. Co-culture of LCL and T cells stimulated with the dendritic cells.

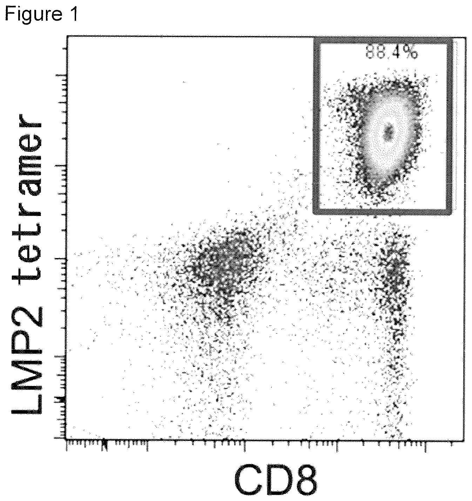

1. The T cells stimulated with the dendritic cells were dispersed in the T cell medium to give a 2×10⁶ cells/mL suspension.
2. 0.5 mL/well of the LCL suspension (2×10⁵ cells/mL) incubated in the presence of the peptide and 0.5 mL/well of T cell suspension (2×10⁶ cells/mL) were added to each well of a 24-well plate (LCL : T cells = 1×10⁵ : 1×10⁶ = 1:10). Simultaneously, the peptide was added to the well to give the final concentration of 100 nM.
3. On day 3, IL-7 (final concentration: 5 ng/mL) and IL-15 (final concentration: 1 ng/mL) were added to the well. The plate was incubated for 2 weeks and the medium was changed every week with the fresh T cell medium supplemented with the cytokines. (1st course of stimulation with peptide-pulsed LCL)
4. LCLs were again incubated in the medium supplemented with 100 nM of the peptide for 2 hours and then, added with the CTLs.
5. On day 3, IL-7 (final concentration: 5ng/mL) and IL-15 (final concentration: 1 ng/mL) were added to the well. The plate was incubated for 2 weeks and the medium was changed every week with the fresh T cell medium supplemented with the cytokines. (2nd course of stimulation with peptide-pulsed LCL)
6. Thus obtained cells were analyzed by flow cytometry and confirmed that more than 80% of CD8 positive T cells were CD8 positive and LMP-2 tetramer positive cells. Results are shown in Figure 1.

### E. Antigen specific killer activity of the LMP2 specific CTLs

1. CFSE-labelled OUN-1 leukemia cells were used as target cells. The labelled cells were dispersed in the T cell medium and incubated in the presence of 1 nM of the LMP2 peptide for 2 hours.
2. LMP2 specific cytotoxic T cells (CD8 positive and LMP-2 tetramer positive cells) expanded under the peptide stimulation and the CFSE-labelled OUN-1 leukemia cells were added to each well of a 96-well round bottom plate at different effector/target cell ratios of 0:1, 1:9, 1:3, 1:1 and 3:1. The cells were incubated in the presence or absence of the peptide. The ratio of Annexin V positive cells to PI (Propidium Iodide) positive cells in the CFSE positive cell fraction were determined to confirm percentage of dead cells among the target cells. Results are shown in Figure 2.
3. Thus prepared LMP2 specific killer T cells were confirmed to have the antigen specific killer activity against the target cells.

### b) Establishment of the LMP2-T-iPS cells

### A. Activation of LMP2 specific CTLs.

1. CD8 positive cells were enriched from the above obtained LMP2 specific CTLs by using MACS beads.
2. The enriched cell population was dispersed in the T cell medium and added with IL-7 (final concentration: 5 ng/mL) and IL-15 (final concentration: 10 ng/mL). Dynabeads Human T-Activator CD3/CD28 was added to give a bead-to-cell ratio of 1:1, and the mixture was incubated for 2 days to activate the CD8 positive cells.

### B. Introduction of the Yamanaka Four factors and SV40 by means of Sendai virus vector.

1. The activated LMP2 specific CTLs were dispersed in the T cell medium, Sendai virus bearing four Yamanaka factors and SV40 was added to the medium and the cell suspension was cultured for 2 days.
2. The obtained cells were washed with the T cell medium and added with the T cell medium supplemented with IL-7 (final concentration: 5 ng/mL) and IL-15 (final concentration: 1 ng/mL). The cells were further cultured for 2 days.
3. After that, all cells were collected and dispersed in the T cell medium supplemented with IL-7 (final concentration: 5ng/mL) and IL-15 (final concentration: 1ng/mL). The cell suspension was seeded on the feeder cells.
4. On day 2, a half of the medium was replaced with the fresh iPS cell medium. After that, a half of the medium was replaced with fresh iPS cell medium every day and the cells were continuously cultured.

### C. Picking up iPS cell colonies from the culture

1. Three weeks after the introduction of the Yamanaka factors, colonies of iPS cells were visually observed.
2. Colonies were mechanically picked up with a 200 µl pipette tip.
3. Several clones were established individually and one of them was used as LMP2-T-iPS cells in the example below. Photograph of the colony of an obtained clone is shown in Figure 3.

### 3) Induction of T cells from the LMP2-iPS cells.

Media used are as follows:

**[Table 2]**

| Medium A: for maintenance of OP9 stromal cells | | |
|---|---|---|
| contents | amount added | final conc. |
| αMEM medium | 500 mL | |
| FCS | 125 mL | 20% |
| penicillin-streptomycin solution* | 6.25 mL | 1% |
| Total | 631.25 mL | |

| | | |
|---|---|---|
| *Mixture of Penicillin (10,000 U/ml) and Streptomycin (10,000 µg/ml). The final concentrations were 100 U/ml and 100 pg/ml, respectively. | | |

**[Table 3]**

| Medium B: for inducing differentiation of T cells | | |
|---|---|---|
| contents | amount added | final conc. |
| αMEM medium | 500 mL | |
| FCS | 125 mL | 20% |
| penicillin-streptomycin solution* | 5 mL | 1% |
| hrIL-7 (stock: 10 µg/ mL) | 315 µL | 5 ng/mL |
| hrFlT-3L (stock: 10 µg/mL) | 315 µL | 5 ng/mL |
| hrSCF (stock: 10 µg/mL) | 630 µL | 10 ng/mL |
| Total | 631.26 mL | |

| | | |
|---|---|---|
| *Mixture of Penicillin (10,000 U/ml) and Streptomycin (10,000 µg/ml). The final concentrations were 100 U/ml and 100 µg/ml, respectively. | | |

**[Table 4]**

| Medium C: for inducing from immature T cells into mature T cells | | |
|---|---|---|
| contents | amount added | final conc. |
| αMEM medium | 500 mL | |
| FCS | 125 mL | 20% |
| penicillin-streptomycin solution* | 5 mL | 1% |
| hrIL-7 (stock: 10 µg/ mL) | 315 µL | 5 ng/mL |
| Total | 630.315 mL | |

| | | |
|---|---|---|
| *Mixture of Penicillin (10,000 U/ml) and Streptomycin (10,000 µg/ml). The final concentrations were 100 U/ml and 100 µg/ml, respectively. | | |

### Preparation of OP9 cells

Six milliliters (6mL) of 0.1% gelatin solution in PBS was added to a 10 cm dish (Falcon) and incubated for 30 minutes at 37°C. The gelatin solution was then removed and 10 ml of medium A was added to the dish. OP9 stromal cells were obtained from a confluent culture and seeded in the dish. Four days after, medium A 10 mL was added to the dish (final amount was 20 mL).

### Induction of hematopoietic progenitor cells from iPS cells

The medium in the OP9 stromal cell culture to be used for the co-culture was aspirated and replaced with fresh medium A. The medium in the iPS cell culture dish was also aspirated and 10 ml of fresh medium A was added. The iPS cell mass was cut with an EZ-passage roller. The cut iPS cell mass was suspended by using a pipetman with a 200 ul tip. The number of the iPS cell clusters was visually counted and approximately 600 iPS cell clusters were seeded on the OP 9 cells. Three or more dishes per clone of iPS cells were used, and when subculturing, the cells in all dishes were once pooled in one dish and then redistributed to the same number of dishes to reduce the disparity between the dishes.

### Day 1: (the medium was replaced)

Whether or not the iPS cell mass adhered to the dish and started to differentiate were confirmed. The cell culture medium was replaced with 20 mL of fresh medium A.

### Day 5: (a half of the medium was replaced)

A half of the cell culture medium was replaced with 10 mL of fresh medium A.

### Day 9: (a half of the medium was replaced)

A half of the cell culture medium was replaced with 10 mL of fresh medium A.

### Day 13: (Induced mesodermal cells were transferred from OP9 cell layer onto OP9/DLL1 cell layer)

Cell culture medium was aspirated to remove and the surface of the cultured cells were washed with HBSS(⁺Mg⁺Ca) to washout the cell culture medium. 10 mL of Collagenase IV 250U in HBSS (+Mg+Ca) solution was added to the dish and incubated for 45 minutes at 37°C.

The collagenase solution was removed by aspiration and the cells were washed with 10 mL of PBS(-). Then, 0.05% trypsin/EDTA solution was added to the dish and the dish was incubated for 20 minutes at 37°C. After the incubation, the sheet like cell aggregates peeled from the bottom of the dish and the cell aggregates were mechanically fragmented to smaller sizes by means of pipetting. Thus treated cells were added with fresh medium A 20 mL and cultured for more 45 minutes at 37°C.

The culture medium containing the floating cells was passed through 100µm mesh and the cells were collected. The cells were then centrifuged at 1200rpm for 7 minutes at 4°C. The obtained pellet was suspended in 10 mL of medium B. One-tenth of the suspension was separated and used for the FACS analysis. The remaining cell suspension was seeded to new dishes containing OP9/DLL1 cells. Cell suspensions obtained from several dishes were pooled and the pooled cells were seeded to the same number of new dishes.

In order to ascertain whether or not hematopoietic progenitor cells were contained in the obtained cells, FACS analysis was carried out using anti-CD34 antibody and anti-CD43 antibody. The results are shown in Fig. 4. Since a sufficient number of cells could be confirmed in the CD34^{low}CD43⁺ cell fraction, it was confirmed that hematopoietic progenitor cells were induced.

### C. Induction of T cells from hematopoietic progenitor cells.

Then, the obtained cells were seeded on OP9/DLL1 cells. In this step, cell sorting of the CD34^{low}CD43⁺ cell fraction was not performed. When this fraction is sorted, the efficiency of differentiation of T cells could be reduced in comparison with the case where sorting was not performed due to the decrease of the cells or damage to the cells by sorting.

During the culturing period, FACS analysis was conducted several times to confirm the differentiation stages. A considerable number of dead cells were observed over the culturing period. Before the FACS analysis, dead cells were eliminated by using, for example, Propidium Iodide (PI) or 7-AAD.

### Day 16: (Cells were subcultured)

The cells loosely adhered to the OP9 cells were dissociated by gently pipetting several times. The cells were passed through a 100 µm mesh and collected in a 50 mL conical tube. The tube was centrifuged at 1200 rpm for 7 minutes at 4°C. The pellet was dispersed in 10 mL of medium B. Thus prepared cell suspension was seeded on the OP9/DLL1 cells.

### Day 23: (Cells were subcultured) Blood cell colonies began to appear.

The cells loosely adhered to the OP9/DLL1 cells were dissociated by gently pipetting several times. The cells were passed through a 100 µm mesh and collected in a 50 mL conical tube. The tube was centrifuged at 1200 rpm for 7 minutes at 4°C. The pellet was dispersed in 10 mL of medium B.

### Day 36: LMP2 tetramer positive cells were confirmed

In order to confirm T cells specific for the LMP2 antigen were induced, the cells on Day 36 were analyzed by FACS with anti CD3 antibody and LMP2 tetramer.

Results are shown in Figure 5. CD3⁺ cells were observed and a part of the cells were differentiated into CD3⁺LMP2 tetramer positive cells.

### D. Induction of mature killer T cells from the immature T cells.

On day 36, LMP2 positive T cells were confirmed with flow cytometry and then, the cells were added with IL-15 so that the cells are differentiated into mature killer T cells or CD8SP cells. The T cells were dispersed in medium C and seeded on the fresh OP9/DLL1 cell layer in each well of a 24-well plate at a density of 3×10⁵ cells/well. IL-15 was added to each well to give final concentration of 10 ng/mL.

### Day 41: Mature killer T cells were observed

Five days after the addition of IL-15, the cells were analyzed with FACS. Result is shown in Figure 6. Mature CD8 single positive cells were observed.

### 4) Antigen-specific killer activity of the re-generated LMP2 specific CTLs

1. CFSE-labelled LCLs were used as target cells. The labelled cells were dispersed in the T cell medium and incubated in the presence of 1 nM of the LMP2 peptide for 2 hours.
2. The regenerated CD8 single positive T cells and the target cells (LCLs) were added to each well of a 96-well round bottom plate at different effector/target cell ratios of 0:1, 1:9, 1:3, 1:1, 3:1, 10:1 and 30:1. The cells were incubated in the presence (p+) or absence (p-) of the peptide. The ratio of Annexin V positive cells to PI (Propidium Iodide) positive cells in the CFSE positive cell fraction were determined to confirm percentage of dead cells among the target cells.
3. Results are shown in Figure 7. Thus prepared LMP2 specific killer T cells were confirmed to have the antigen specific killer activity against the target cells.

### 5) Natural killer cell-like activity of the re-generated LMP2 specific CTLs

1. K562 cell line that does not express HLA on the cell surface (to determine alloreactivity) and peripheral blood mononuclear cells of the healthy volunteer A (MA p-) (to determine auto reactivity) were used as target cells. Those cells were labelled with CFSE and suspended in the T cell medium.
2. The regenerated CD8T cells and the target cells were added to each well of a 96-well round bottom plate at different effector/target cell ratios of 0:1, 1:9, 1:3, 1:1, and 3:1. The cells were incubated and the ratio of Annexin V positive cells to PI (Propidium Iodide) positive cells in the CFSE positive cell fraction were determined to confirm percentage of dead cells among the target cells.
3. Results are shown in Figure 8. The LMP2 specific killer T cells did not kill the autologous PBMC (MA p-) but showed high killer activity against K562 cells. This result support that the LMP2 specific killer T cells have natural-killer cell like activity.

### 6) Antigen specific killer activity of the re-generated LMP2 antigen specific CTLs

1. CFSE labelled LCLs were used as target cells. The cells were suspended in the T cell medium and incubated in the presence of the LMP2 peptide for 2 hours.
2. The regenerated CD8 single positive T cells (Re-generated LMP2-CTL#1 and the target cells (LCLs) were added to each well of a 96-well round bottom plate at different effector/target cell ratios of 0:1, 1:9, 1:3, 1:1, 3:1 and 9:1. The cells were incubated in the presence of various concentrations of LMP2 peptide or absence of the peptide. After 6 hours incubation, the ratio of Annexin V positive cells to PI (Propidium Iodide) positive cells in the CFSE positive cell fraction were determined to confirm percentage of dead cells among the target cells. Results are shown in Figure 9.
3. The regenerated LMP2-CTL#1 showed high antigen specific cytotoxic activity against the peptide-loaded LCLs.

### 7) The sequences of TCR alpha and beta chains of clone LMP2#1 were determined

TCRα chain: TRAV26-1*01-CIVTRFYTDKLIF-TRAJ34*01 (SEQ ID NO: 3)
TCRβ chain: TRBV14*02-CASSSPGSRPYNEQFF-TRBJ2-1*01 (SEQ ID NO: 4)

### [EXAMPLE 2]

LMP2 peptide specific CTLs were induced according to the procedure of Example 1 from a healthy volunteer other than healthy volunteer A from whom PBMC were obtained in Example 1. T-iPS cells (clone LMP2#13) were established from the CTL and then, the T-iPS cells were differentiated into CD8 single positive T cells (re-generated LMP2-CTL#13). The LMP2 peptide used in Example 1 was also used in this example. The antigen specific killer activity of the re-generated CTL cells against the peptide-loaded LCL cells as target cells was determined. Result is shown in Example 10. The healthy volunteer in Example 2 had previously been infected with EB virus and was EBNA antibody positive, and had HLA-A*02:10/24:02 ; B*07:02/40:06; C*07:02/08:01; DRB1*04:05/04:05.

The regenerated LMP2-CTL#13 showed high antigen specific cytotoxicity against the peptide-loaded LCLs.

The amino acid sequences of TCR alpha and beta chains of clone LMP2#13 were determined
TCRα chain: TRAV25*01-CAGERGSTLGRLYF-TRAJ18*01 (SEQ ID NO: 5)
TCRβ chain: TRBV7-9*03-CASSPLSTGNYEQYF-TRBJ2-7*01 (SEQ ID NO: 6)

### [EXAMPLE 3]

WT1 antigen specific cytotoxic T cells were induced from peripheral blood of a healthy volunteer, and T-iPS cells (clone WT#9) were established from the CTL. Then, WT1 antigen specific mature T cells (re-generated WT1-CTL(#9)) were induced from the T-iPS cells.

This example comprises the following steps:
1) Amplification of WT1 antigen specific CTLs
2) Establish of WT1-T-iPS cells
3) Induction of CD8 single positive T cells (CTLs) from the WT1-T-iPS cells.
4) Confirmation of antigen specific killer activity of the re-generated WT1-CTL obtained in step 3).

### 1) Amplification of WT1 antigen specific CTL

i) The medium used is as follows.

**[Table 5]**

| Medium for T cells (T cell medium): | | |
|---|---|---|
| | Amount | Final conc. |
| RPMI | 45ml | |
| human AB serum | 5ml | 10% |
| Total | 50ml | |

ii) The WT1 antigen peptide used is as follows.
WT1 modified form: CYTWNQMNL (SEQ ID NO: 2) Cancer Immunol. Immunothera. 51: 614 (2002))
Both WT1 peptide and WT1 tetramer used below were the modified form.
iii) The LCL (Lymphoblastoid cell line) used is as follows.

The LCL having HLA-A2402 which had been established from a healthy volunteer in the Department of Hematology and Oncology, Graduate School of Medicine, Kyoto University, Kyoto, Japan was used.

### A. Isolation of T cells from human peripheral blood and stimulation of the cells with the peptide

1. Peripheral blood was obtained from a healthy volunteer. Monocytes were purified from the blood by using Ficoll and dispersed in the T cell medium. The healthy volunteer has HLA-A*02:01/24:02; B*15:01/15:11; C*03:03/08:01; DRB1*12:01/12:02.
2. The cell suspension was added to each well of a 96-well round bottom plate in a density of 2.5×10⁵ cells/mL/well, and the peptide was added to give the final concentrations of 10µm.
3. On day 3, IL-2 (final concentration: 12.5 U/mL), IL-7 (final concentration: 5ng/mL) and IL-15 (final concentration: 1 ng/mL) were added to the well. The plate was incubated for 2 weeks and the medium was changed every week with the fresh T cell medium supplemented with the cytokines.

### B. Addition of the peptide to LCLs.

1. LCLs were collected from the culture and irradiated at a dose of 35Gy.
2. The irradiated cells were suspended in the T cell medium to give a 5×10⁵ cells/mL suspension.
3. The peptide 100nM was added to the suspension and incubated for 2 hours.
4. The LCLs were collected and washed with the T cell medium and then, dispersed in the T cell medium to give a 2×10⁵ cells/mL suspension.

### C. Co-culture of LCL pulsed with the peptide and T cells.

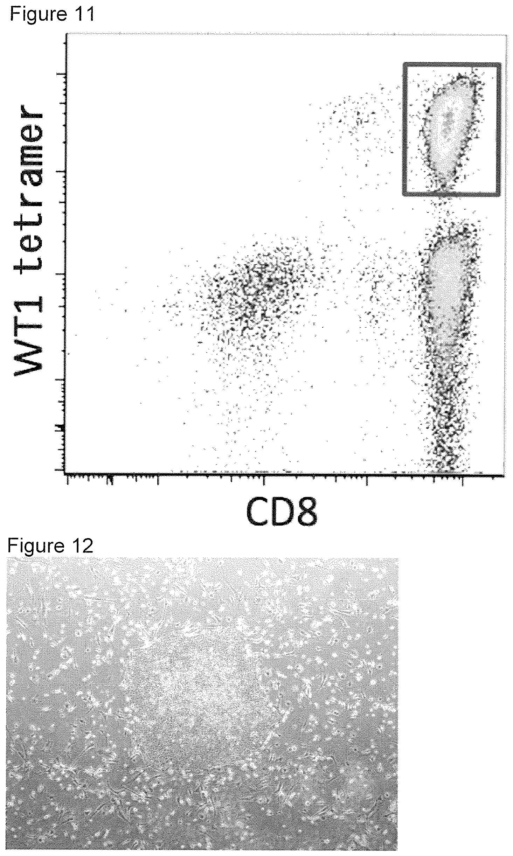

1. The peptide stimulated T cells were collected when they were incubated for two weeks after the peptide stimulation, washed and then dispersed in the T cell medium to give 2×10⁶ cells/mL suspension. A small part of the T cell suspension was separated for the flow cytometer analysis.
2. A LCL suspension (2×10⁵ cells/mL) that had been incubated in the presence of the peptide 0.5 mL/well and the T cell suspension (2×10⁶ cells/mL) 0.5 mL/well were added to each well of a 24 well plate. (LCLs: T cells=1×10⁵ : 1×10⁶=1:10).
3. On day 3, IL-2 (final concentration: 12.5 U/mL), IL-7 (final concentration: 5 ng/mL) and IL-15 (final concentration: 1 ng/mL) were added to each well. The plate was incubated for 2 weeks and the medium was changed every week with the fresh T cell medium supplemented with the cytokines. (1st course of stimulation with peptide-pulsed LCL)
4. LCLs were again incubated in the medium supplemented with 100 nM of the peptide for 2 hours and then, added with the CTLs.
5. On day 3, IL-2 (final concentration: 12.5 U/mL), IL-7 (final concentration: 5 ng/mL) and IL-15(final concentration: 1 ng/mL) were added to each well. The plate was incubated for 2 weeks and the medium was changed every week with the fresh T cell medium supplemented with the cytokines. (2nd course of stimulation with peptide-pulsed LCL)
6. LCLs were again incubated in the medium supplemented with 100 nM of the peptide for 2 hours and then, added with the CTLs.
7. On day 3, IL-2 (final concentration: 12.5 U/mL), IL-7 (final concentration: 5 ng/mL) and IL-15(final concentration: 1 ng/mL) were added to each well. The plate was incubated for 2 weeks and the medium was changed every week with the fresh T cell medium supplemented with the cytokines. (3rd course of stimulation with peptide-pulsed LCL)
8. Thus obtained cells were analyzed by flow cytometry. The result is shown in Figure.11. It was confirmed that more than 60% of the CD8 positive T cells were CD8 positive and WT1 tetramer positive cells.

### 2) Establish of the WT1-T-iPS cells

A. Activation of WT1 specific CTLs.
1. CD8 positive cells were enriched from the above obtained WT1 specific CTLs using MACS beads.
2. The enriched cell population was dispersed in the T cell medium and added with IL-2 (final concentration: 12.5 U/mL), IL-7 (final concentration: 5 ng/mL) and IL-15 (final concentration: 1 ng/mL). Dynabeads Human T-Activator CD3/CD28 was added to give a bead-to-cell ratio of 1:1, and the mixture was incubated for 2 days to activate the CD8 positive cells.

### B. Introduction of the Yamanaka Four factors and SV40 by means of Sendai virus vector.

1. The activated WT1 specific CTLs were dispersed in the T cell medium, Sendai virus bearing four Yamanaka factors and SV40 was added to the medium and the cell suspension was cultured for 2 days.
2. The obtained cells were washed with the T cell medium and added with the T cell medium supplemented with IL-2 (final concentration: 12.5 U/mL), IL-7 (final concentration: 5 ng/mL) and IL-15 (final concentration: 1 ng/mL). The cells were further cultured for 2 days.
3. After that, all cells were collected and dispersed in the T cell medium containing no cytokine. The cell suspension was seeded on the feeder cells.
4. On day 2, a half of the medium was replaced with the fresh iPS cell medium. After that, a half of the medium was replaced with the fresh iPS cell medium every day and the cells were continuously cultured.

### C. Picking up iPS cell colonies from the culture

1. Three weeks after the introduction of the Yamanaka factors, colonies of iPS cells were visually observed.
2. Colonies were mechanically picked up with a 200 µl pipette tip.
3. Several clones were established individually. Photograph of the colony of an obtained clone is shown in Figure 12.

### 3) Induction of T cells from the WT1-T-iPS cells.

Media used are as follows:

**[Table 6]**

| Medium A: for maintenance of OP9 stromal cells | | |
|---|---|---|
| contents | amount added | final conc. |
| αMEM medium | 500 mL | |
| FCS | 125 mL | 20% |
| penicillin-streptomycin solution* | 6.25 mL | 1% |
| Total | 631.25 mL | |

| | | |
|---|---|---|
| *Mixture of Penicillin (10,000 U/ml) and Streptomycin (10,000 µg/ml). The final concentrations were 100 U/ml and 100 µg/ml, respectively. | | |

**[Table 7]**

| Medium B: for inducing differentiation of T cells | | |
|---|---|---|
| contents | amount added | final conc. |
| αMEM medium | 500 mL | |
| FCS | 125 mL | 20% |
| penicillin-streptomycin solution* | 5 mL | 1% |
| hrIL-7 (stock: 10 µg/ mL) | 315 µL | 5 ng/mL |
| hrFlT-3L (stock: 10 µg/mL) | 315 µL | 5 ng/mL |
| hrSCF (stock: 10 µg/mL) | 630 µL | 10 ng/mL |
| Total | 631.26 mL | |

| | | |
|---|---|---|
| *Mixture of Penicillin (10,000 U/ml) and Streptomycin (10,000 µg/ml). The final concentrations were 100 U/ml and 100 µg/ml, respectively. | | |

### Preparation of OP9 cells

Six milliliters (6mL) of 0.1% gelatin solution in PBS was added to a 10 cm dish (Falcon) and incubated for 30 minutes at 37°C. OP9 stromal cells were detached from a confluent culture dish with trypsin/EDTA solution and about 1/4 of the obtained cells were added to the gelatin coated 10cm cell culture dish. 10 mL of medium A was added to the cell culture dish. Four days after, medium A 10 mL was added to the dish (final amount was 20 mL).

### Induction of hematopoietic progenitor cells from iPS cells

The medium in the OP9 stromal cell culture to be used for the co-culture was aspirated and replaced with fresh medium A. The medium in the iPS cell culture dish was also aspirated and 10 ml of fresh medium A was added. The iPS cell mass was cut with an EZ-passage roller. The cut iPS cell mass was suspended by means of a pipetman with a 200 ul tip. The number of the iPS cell clusters was visually counted and approximately 600 iPS cell clusters were seeded on the OP 9 cells. Three or more dishes per clone of iPS cells were used, and when subculturing, the cells in all dishes were once pooled in one dish and then redistributed to the same number of dishes to reduce the disparity between the dishes.

### Day 1: (the medium was replaced)

Whether or not the iPS cell mass adhered to the dish and started to differentiate were confirmed. The cell culture medium was replaced with 20 mL of fresh medium A.

### Day 5: (a half of the medium was replaced)

A half of the cell culture medium was replaced with 10 mL of fresh medium A.

### Day 9: (a half of the medium was replaced)

A half of the cell culture medium was replaced with 10 mL of fresh medium A.

### Day 13: (Induced mesodermal cells were transferred from OP9 cell layer onto OP9/DLL1 cell layer)

Cell culture medium was aspirated to remove and the surface of the cultured cells were washed with HBSS (⁺Mg⁺Ca) to washout the cell culture medium. 10 mL of Collagenase IV 250U in HBSS (+Mg+Ca) solution was added to the dish and incubated for 45 minutes at 37°C.

The collagenase solution was removed by aspiration and the cells were washed with 10 mL of PBS(-). Then, 0.05% trypsin/EDTA solution was added to the dish and the dish was incubated for 20 minutes at 37°C. After the incubation, the sheet like cell aggregates peeled from the bottom of the dish and the cell aggregates were mechanically fragmented to smaller sizes by means of pipetting. Thus treated cells were added with fresh medium A 20 mL and cultured for more 45 minutes at 37°C. The culture medium containing the floating cells was passed through 100µm mesh and the cells were collected. The cells were then centrifuged at 1200rpm for 7 minutes at 4°C. The obtained pellet was suspended in 10 mL of medium B. One-tenth of the suspension was separated and used for the FACS analysis. The remaining cell suspension was seeded to new dishes containing OP9/DLL1 cells. Cell suspensions obtained from several dishes were pooled and the pooled cells were seeded to the same number of new dishes.

In order to ascertain whether or not hematopoietic progenitor cells were contained in the obtained cells, FACS analysis was carried out using anti-CD34 antibody, anti-CD43 antibody. The results are shown in Fig. 4. Since a sufficient number of cells could be confirmed in the CD34^{low}CD43⁺ cell fraction, it was confirmed that hematopoietic progenitor cells were induced.

### C. Induction of T cells from hematopoietic progenitor cells.

Then, the obtained cells were seeded on OP9/DLL1 cells. In this step, cell sorting of the CD34^{low}CD43⁺ cell fraction was not performed. When this fraction is sorted, the efficiency of differentiation of T cells could be reduced in comparison with the case where sorting is not performed due to the decrease of the cells or damage to the cells by sorting.

### Day 16: (Cells were subcultured)

The cells loosely adhered to the OP9 cells were gently dissociated by pipetting several times. The cells were passed through a 100 µm mesh and collected in a 50 mL conical tube. The tube was centrifuged at 1200 rpm for 7 minutes at 4°C. The pellet was dispersed in 10 mL of medium B. Thus prepared cells were seeded on the OP9/DLL1 cells.

### Day 23: (Cells were subcultured) Blood cell colonies began to appear.

The cells loosely adhered to the OP9/DLL1 cells were gently dissociated by pipetting several times. The cells were passed through a 100 µm mesh and collected in a 50 mL conical tube. The tube was centrifuged at 1200 rpm for 7 minutes at 4°C. The pellet was dispersed in 10 mL of medium B.

### Day 36: WT1 tetramer positive T cells were confirmed

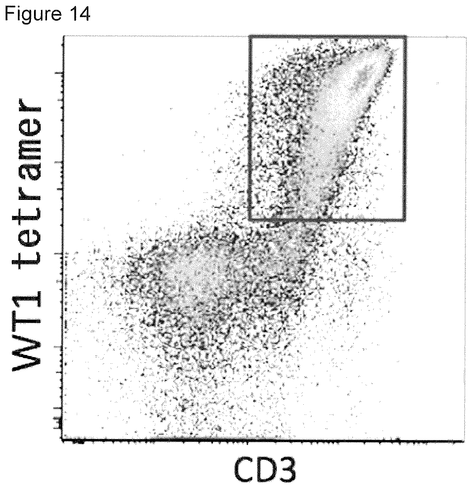

In order to confirm T cells specific for WT1 antigen were induced, the cells on Day 36 were analyzed by FACS with anti CD3 antibody and WT1 tetramer. Results are shown in Figure 14. CD3⁺ cells were observed and a most part of the cells were differentiated into CD3⁺WT1 tetramer positive cells.

As shown above, the T cells regenerated from the T-iPS cells were confirmed to exhibit the same antigen specificity as the original T cells. Further, thus regenerated T cells expressed the surface antigen that were observed in mature T cells and therefore, had the well matured functions.

The amino acid sequences of TCR alpha and beta chains of clone WT1#9 were determined by the conventional method.
TCRα chain: TRAV12-3*01-CAMIRGNTDKLIF-TRAJ34*01 (SEQ ID NO: 7)
TCRβ chain: TRBV5-5*02-CASSFPSYEQYF-TRBJ2-7*01 (SEQ ID NO: 8)

### 4) Antigen specific killer activity of the T cells re-generated from the WT1-T-iPS cells.

1. CFSE labelled LCLs were used as target cells. The cells were suspended in the T cell medium and incubated in the presence of the WT1 peptide (SEQ ID NO: 2) for 2 hours.
2. The regenerated CD8 single positive T cells and the target cells (LCLs) were added to each well of a 96-well round bottom plate at different effector/target cell ratios of 0:1, 1:3, 1:1, 3:1 and 9:1. The cells were incubated in the presence of various concentrations of the peptide or absence of the peptide for 6 hours. After the incubation, the ratio of Annexin V positive cells to PI (Propidium Iodide) positive cells in the CFSE positive cell fraction were determined to confirm percentage of dead cells among the target cells.

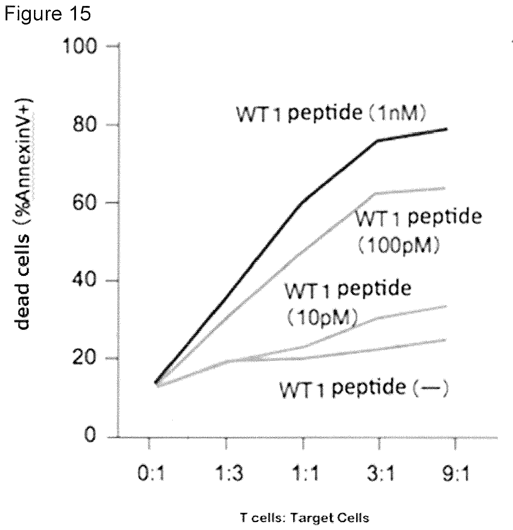

The regenerated WT1-CTL#9 showed high antigen specific cytotoxicity against the peptide-loaded LCLs (Figure 15).

### [EXAMPLE 4]

WT1 peptide specific CTL cells were induced according to the procedure of Example 3 from the same healthy volunteer from whom PBMC were obtained in Example 3. T-iPS cells (clone WT1#3-3) were established from the CTL and then, the T-iPS cells were differentiated into CD8 single positive T cells (re-generated WT1-CTL#3-3). The WT1 peptide used in Example 3 was also used in this example. The antigen specific killer activity of the re-generated CTLs against the LCLs loaded with the peptide as target cells was examined.

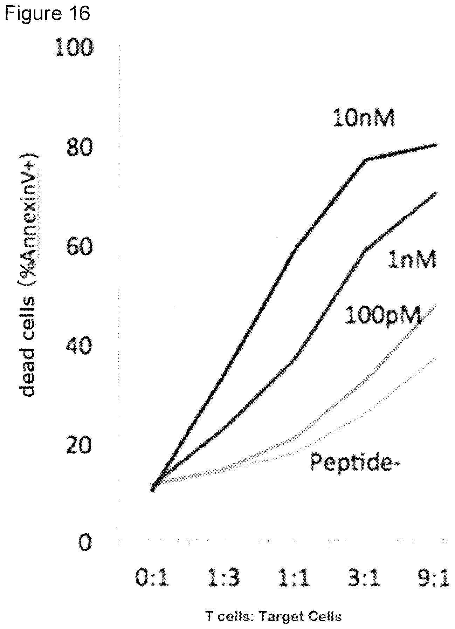

Results are shown in Figure 16. The re-generated WT1-CTL#3-3 showed high antigen specific killing activity against the peptide-loaded LCLs.

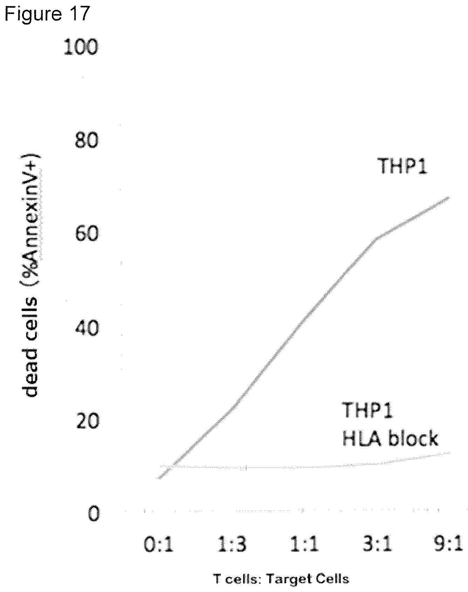

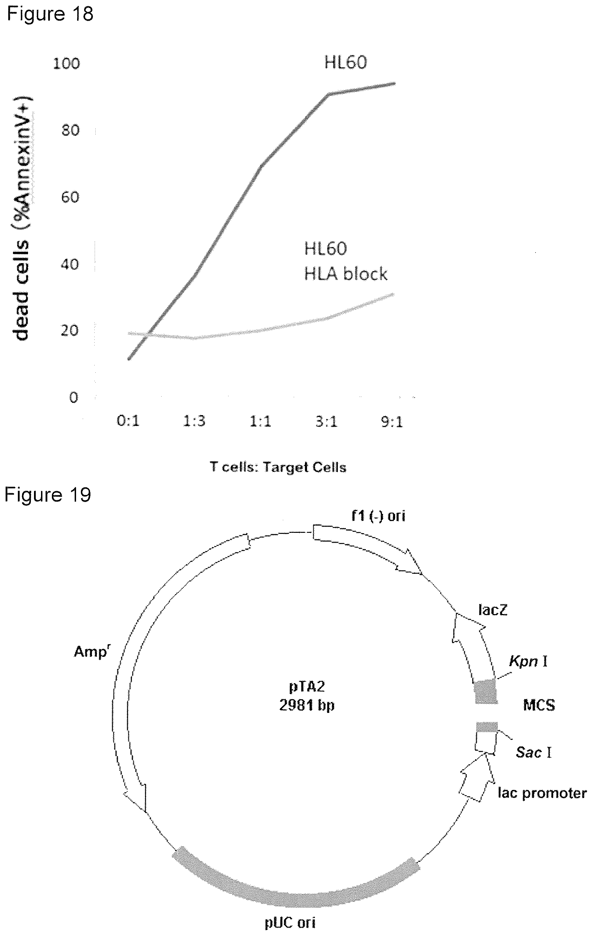

Cytotoxic activities of the re-generated WT1-CTL#3-3 against the leukemia cell lines THP1 and HL60 that expresses WT1 antigen were examined. In addition, whether or not anti-HLA class I antibody could block the cytotoxic activity was examined. The results are shown in Figures 17 and 18.

The re-generated WT1-CTLs#3-3 were cytotoxic against both cell lines THP-1 and HL60 that express WT1 antigen. The cytotoxic activities were completely blocked by the anti-HLA class I antigen. Based on the results, the re-generated WT1-CTL(#3-3) kill the leukemia cells in the antigen specific manner.

The sequences of TCR alpha and beta chains of clone WT1#3-3 were determined
TCRα chain: TRAV12-1*01-CVVRGGGFKTIF-TRAJ9*01 (SEQ ID NO: 9)
TCRβ chain: TRBV20-1*01-CSARAGTGGANVLTF-TRBJ2-6*01 (SEQ ID NO: 10)

### [EXAMPLE 5]

### Establishment of iPS cells with homozygous HLA into which a class I restricted WT1 antigen specific TCR is introduced

The original iPS cells were established from peripheral blood mononuclear cells of a healthy donor in Department of Immunology, Institute for Frontier Medicinal Sciences, Kyoto University, Kyoto, Japan in the same procedure in Example 2. The iPS cell line has homozygous HLAs.

Class I restricted WT1 specific TCR genes were cloned from Clone WT1#9 and Clone WT1#3-3 in Department of Immunology, Institute for Frontier Medicinal Sciences, Kyoto University, Kyoto, Japan.

### Cloning of WT1 specific TCR genes by means of the Rapid Amplification of cDNA ends (RACE)

a WT1 specific CTL clone or a CTL clone induced from WT1-T-iPS cell was amplified and RNAs of the cells were obtained. Full length cDNA was obtained by using SMARTer RACE cDNA amplificatioin kit (Clontech Laboratories, Inc.) and was used as a template. TCR genes were amplified by using a primer targeting the 3'-end of the TCRα chain: CACAGGCTGTCTTACAATCTTGCAGATC (SEQ ID NO: 11) or of TCRβ chain: CTCCACTTCCAGGGCTGCCTTCA (SEQ ID NO: 12) or TGACCTGGGATGGTTTTGGAGCTA(SEQ ID NO: 13) to obtain double stranded WT1-TCR cDNA. Thus obtained double stranded cDNA was inserted into pTA2 vector (TOYOBO, see Figure 19) and introduced into a cell line. The specificity of the WT1 TCR were evaluated using the transfected cells.

### 2) Preparation of lentiviral vector incorporated with WT1-TCR

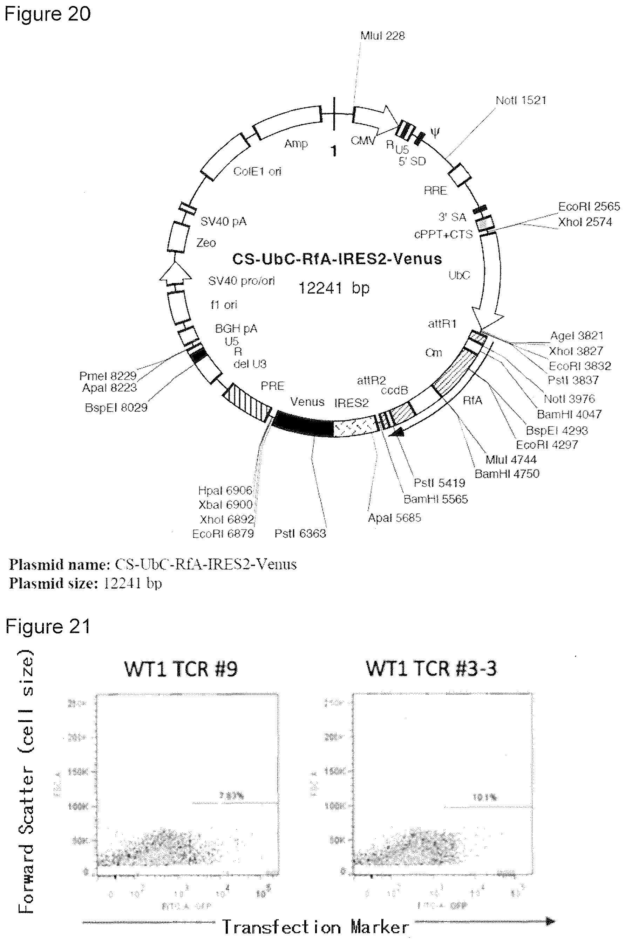

CS-UbC-RfA-IRES2-Venus vector (Figure 20) was obtained from Subteam for Manipulation of Cell Fate, RIKEN BioResource Center, Tsukuba, Ibaraki, Japan. WT-TCR gene was incorporated in the vector with the Gateway system to give CS-UbC-RfA-IRES2-Venus/WT1-TCR.

### 3) Preparation for supernatant of WT1-TCR incorporated lentivirus

CS-UbC-RfA-IRES2-Venus/WT1-TCR was introduced into LentiX-293T packaging cells with X-treamGENE9 (Roche). The medium was exchanged on the next day and on day 2, the culture supernatant was collected and used as lentiviral supernatant.

### 4) Establishment of WT1-TCR transduced T-iPS cells

LMP2-T-iPS cells were treated with TrypLE Select (Life Technologies) to give completely single-cell suspension. The suspension was centrifuged and the pellet was dispersed by the lentiviral supernatant, and then, the obtained suspension was centrifuged at 3000rpm at 32°C for one hour so that lentivirus infects and then, WT1-TCR was introduced into the LMP2-T-iPS cells.

2. A single-cell suspension of the iPS cells transfected with the genes was analyzed by flow cytometry. The results are shown in Figure 21. It was confirmed that WT1 specific TCR genes derived from Clone #9 and Clone #3-3 were duly introduced in the iPS cells.

## Claims

1. A method for inducing T cells for use in a cell-based immunotherapy, comprising the steps of:
(1) providing human pluripotent stem cells bearing a T cell receptor specific for a WT1 antigen, and
(2) inducing T cell progenitors or mature T cells from the pluripotent stem cells provided in step (1).

2. The method according to claim 1, wherein the human pluripotent stem cells bearing a T cell receptor specific for a WT1 antigen are obtained by inducing pluripotent stem cells from a human T cell specific for the WT1 antigen.

3. The method according to claim 2, wherein the human T cell specific for a WT1 antigen is obtained from a person who is not the subject to be treated by the cell-based immunotherapy, and is suffered from or had previously been suffered from a WT1 expressing cancer.

4. The method according to claim 2, wherein the human T cell specific for a WT1 antigen is obtained from a person who is not the subject to be treated by the cell-based immunotherapy, and has never been suffered from a WT1 expressing cancer.

5. The method according to claim 1, wherein the human pluripotent stem cells bearing T cell receptor specific for a WT1 antigen are obtained by introducing genes encoding the T cell receptor specific for the WT1 antigen into iPS cells.

6. A method for inducing T cells for use in a cell-based immunotherapy, comprising the steps of:
(1) providing human pluripotent stem cells bearing a T cell receptor specific for an Epstein-Barr virus associated antigen, and
(2) inducing T cell progenitors or mature T cells from the pluripotent stem cells provided in step (1).

7. The method according to claim 6, wherein the human pluripotent stem cells bearing a T cell receptor specific for an Epstein Barr virus associated antigen are obtained by inducing pluripotent stem cells from a human T cell specific for the Epstein Barr virus associated antigen.

8. The method according to claim 7, wherein the human T cell specific for an Epstein Barr virus associated antigen is obtained from a person who is not the subject to be treated by the cell-based immunotherapy, and is suffered from or had previously been suffered from an Epstein Barr virus associated disease.

9. The method according to claim 7, wherein the human T cell specific for an Epstein Barr virus associated antigen is obtained from a person who is not the subject to be treated by the cell-based immunotherapy, and has never been suffered from an Epstein Barr virus associated disease.

10. The method according to claim 6, wherein the human pluripotent stem cells bearing a T cell receptor specific for an Epstein Barr virus associated antigen are obtained by introducing genes encoding the T cell receptor specific for the Epstein Barr virus antigen into iPS cells.

11. The method according to any one of claims 7-9, wherein the human T cell specific for an Epstein Barr virus antigen is a T cell specific for a LMP2 antigen.

12. The method according to any one of claims 1-11, wherein the human pluripotent stem cells bearing a T cell receptor specific for an antigen are pluripotent stem cells induced from a cell of a person having HLA phenotypes that are completely or partially identical to the HLA phenotypes of the subject to be treated by the cell-based immunotherapy.

13. The method according to claim 12, wherein the human pluripotent stem cells bearing a T cell receptor specific for the antigen are pluripotent stem cells induced from a cell of a person homozygous for HLA haplotype that matches at least one of HLA haplotypes of the subject to be treated.

14. The method according to any one of claims 1-13, further comprises the step of co-culturing the T cell progenitors or mature T cells induced from the pluripotent stem cells with the lymphocytes of the subject to be treated by the cell based immunotherapy to verify that the T cells are not allogenicaly reactive against the subject.

15. The method according to any one of claims 1-14, wherein the human pluripotent stem cells are human iPS cells.

16. The method according to any one of claims 1-15, wherein the cell-based immunotherapy is for the treatment of a disease selected from the group consisting of a cancer, an infectious disease, an autoimmune disease and an allergy.

17. The method according to claim 16, wherein the cancer is an EB virus relating cancer.

18. The method according to claim 16, wherein the infectious disease is an EB virus associated disease.

19. The method according to claim 16, wherein the cancer expresses WT1 gene.
